# EUROPÄISCHE PATENTANMELDUNG

(11) **EP 0 726 273 A1**
(43) Veröffentlichungstag der Anmeldung: **14.08.1996**
(21) Anmeldenummer: 96100801.8
(22) Anmeldetag: 20.01.1996
(51) Int. Cl.: C07H 17/065, A61K 31/70, C08B 37/00, A61K 31/715, A61K 7/00, A61K 7/48, C11B 5/00

(54) **Tocopherylglycoside, ihre Herstellung sowie ihre Verwendung als Tenside, als Antioxidantien sowie als der Zellalterung vorbeugender Wirkstoff in kosmetischen oder pharmazeutischen Zubereitungen**

(30) Priorität: 10.02.1995 DE 19504398
(71) Anmelder: Beiersdorf Aktiengesellschaft, D-20245 Hamburg (DE)
(72) Erfinder: Schneider, Günther, Dr., D-20253 Hamburg (DE); Thiem, Joachim, Prof. Dr., D-22391 Hamburg (DE); Lahmann, Martina, D-22303 Hamburg (DE)

(57) **Zusammenfassung**

Tocopherylglycoside der allgemeinen Formel 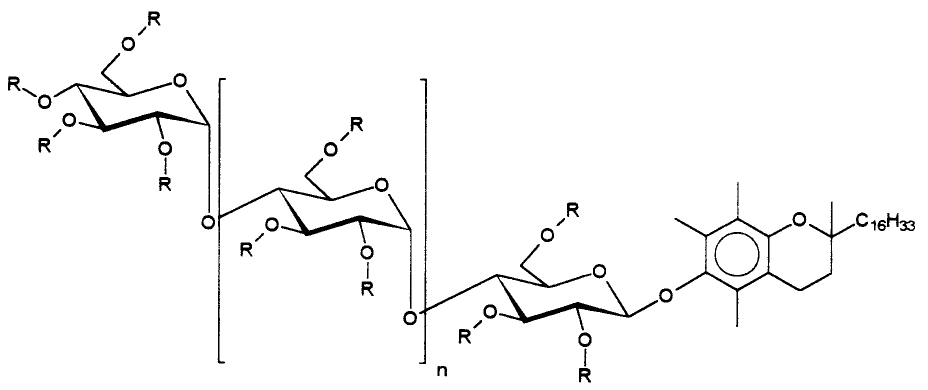 oder der allgemeinen Formel 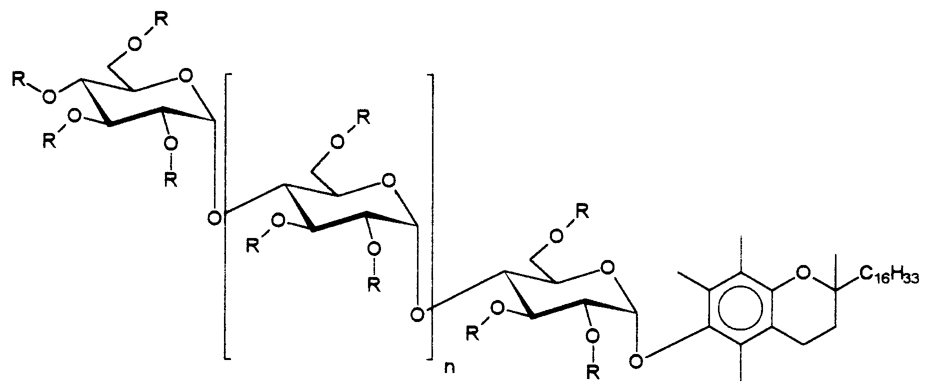 wobei n Werte von 0 - 6 annehmen kann und wobei R stellvertretend steht für die Gruppe bestehend aus den Resten H, verzweigtem und unverzweigtem Alkyl von 1 - 18 C-Atomen, verzweigtem und unverzweigtem Acyl von 1 - 18 C-Atomen, und wobei R innerhalb eines Moleküls in allen Positionen der Glycosylgruppen gleich sein kann, aber auch innerhalb eines Moleküls unterschiedliche Bedeutung annehmen kann, dergestalt, daß innerhalb eines Moleküls beliebige Kombinationen der stellvertretenden Reste gewählt werden dürfen.

## Beschreibung

Die vorliegende Erfindung betrifft neue Wirkstoffe, deren Herstellung sowie deren Verwendung auf dem Gebiete der kosmetischen sowie der pharmazeutischen Dermatologie. Insbesondere betrifft die vorliegende Erfindung Wirkstoffe und kosmetische oder dermatologische Zubereitungen, solche Wirkstoffkombinationen enthaltend. Insbesondere betrifft die vorliegende Erfindung kosmetische Zubereitungen mit einem Gehalt an die Haut, aber auch die Zubereitungen selbst gegen Oxidationsprozesse schützenden Substanzen. Ferner betrifft die Erfindung Wirkstoffe, welche Tensideigenschaften besitzen.

Stabile kosmetische Emulsionen enthalten grenzflächenaktive Stoffe, sogenannte Tenside bzw. Emulgatoren. Diese setzen die Grenzflächen zwischen den Phasen herab und bilden an der Phasengrenze Öl/Wasser Grenzflächenfilme aus. Dadurch wird dem irreversiblen Zusammenfließen der dispergierten Phase entgegengewirkt.

Leistungsfähige Emulgatoren zeichnen sich daher durch eine sehr gute Emulgier-, Solubilisierungs- sowie Dispergierungsfähigkeit aus. Es ist in hohem Maße wünschenswert, daß solche Substanzen keine Hautreizung auslösen. Die Auswahl solcher dem Stande der Technik bekannten Substanzen ist begrenzt. Es war daher eine Aufgabe der vorliegenden Erfindung, den Stand der Technik in dieser Hinsicht zu bereichern.

Die vorliegende Erfindung betrifft ferner Antioxidantien, bevorzugt solche, welche in hautpflegenden kosmetischen oder dermatologischen Zubereitungen eingesetzt werden. Insbesondere betrifft die Erfindung auch kosmetische und dermatologische Zubereitungen, solche Antioxidantien enthaltend. In einer bevorzugten Ausführungsform betrifft die vorliegende Erfindung kosmetische und dermatologische Zubereitungen zur Prophylaxe und Behandlung kosmetischer oder dermatologischer Zellveränderungen, insbesondere Hautveränderungen wie z.B. der Hautalterung, insbesondere der durch oxidative Prozesse hervorgerufenen Zellalterung, insbesondere der Hautalterung.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutz gegen UVB-Strahlung sind zahlreiche Verbindungen bekannt, bei denen es sich um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, des sogenannten UVA-Bereich, ist es wichtig, Filtersubstanzen zur Verfügung zu haben, da dessen Strahlen Reaktionen bei lichtempfindlicher Haut hervorrufen können. Es ist erwiesen, daß UVA-Strahlung zu einer Schädigung der elastischen und kollagenen Fasern des Bindegewebes führt, was die Haut vorzeitig altern läßt, und daß sie als Ursache zahlreicher phototoxischer und photoallergischer Reaktionen zu sehen ist. Der schädigende Einfluß der UVB-Strahlung kann durch UVA-Strahlung verstärkt werden.

Zum Schutz gegen die Strahlen des UVA-Bereichs werden daher gewisse Derivate des Dibenzoylmethans verwendet.

Die UV-Strahlung kann aber auch zu photochemischen Reaktionen führen, wobei dann die photochemischen Reaktionsprodukte in den Hautmetabolismus eingreifen.

Vorwiegend handelt es sich bei solchen photochemischen Reaktionsprodukten um radikalische Verbindungen, beispielsweise Hydroxyradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Ferner zählt UV-Strahlung zur ionisierenden Strahlung. Es besteht also das Risiko, daß auch ionische Spezies bei UV-Exposition entstehen, welche dann ihrerseits oxidativ in die biochemischen Prozesse einzugreifen vermögen.

Um diesen Reaktionen vorzubeugen, können den kosmetischen bzw. dermatologischen Formulierungen zusätzliche Antioxidantien und/oder Radikalfänger einverleibt werden.

Es ist bereits vorgeschlagen worden, Vitamin E, eine Substanz mit bekannter antioxidativer Wirkung in Lichtschutzformulierungen einzusetzen, dennoch bleibt auch hier die erzielte Wirkung wert hinter der erhofften zurück.

In den vergangenen Jahren wurden viele verschiedene Derivate und Darreichungsformen von Vitamin E entwickelt, um dessen Verfügbarkeit zu erhöhen. Solche Derivate stellen oft Tocopherylester dar, welche jedoch den Nachteil aufweisen, daß ihre antioxidative Wirksamkeit deutlich geringer ist als das underivatisierte Vitamin E. Zudem sind solche Derivate meist nur in Fetten oder organischen Lösemitteln löslich sind. Tocopherylphosphate und -ethoxylate, welche der Synthese zugängig sind, haben andere Nachteile.

Es war eine Aufgabe der vorliegenden Erfindung, Tocopherylderivate mit vorteilhaften antioxidativen Eigenschatten sowie für die kosmetische und pharmazeutische Galenik guter Löslichkeit zu entwickeln.

Aufgabe der Erfindung war es auch, kosmetische, dermatologische und pharmazeutische Wirkstoffe und Zubereitungen sowie Lichtschutzformulierungen zu schaffen, die zur Prophylaxe und Behandlung lichtempfindlicher Haut, insbesondere Photodermatosen, bevorzugt PLD dienen.

Weitere Bezeichnungen für die polymorphe Lichtdermatose sind PLD, PLE, Mallorca-Akne und eine Vielzahl von weiteren Bezeichnungen, wie sie in der Literatur (z.B. A. Voelckel et al, Zentralblatt Haut- und Geschlechtskrankheiten (1989), 156, S.2), angegeben sind.

Gegenstand der Erfindung sind auch die Gemische der Wirkstoffe und Zubereitungen damit.

Weiterhin betrifft die vorliegende Erfindung Wirkstoffe und Zubereitungen, solche Wirkstoffe enthaltend, zur kosmetischen und dermatologischen Behandlung oder Prophylaxe erythematöser, entzündlicher, allergischer oder autoimmunreaktiver Erscheinungen, insbesondere Dermatosen. Ferner betrifft die Erfindung die Verwendung solcher Wirkstoffe und Zubereitungen, solche Wirkstoffe enthaltend, zur Immunstimulation der Haut, dabei vorteilhaft auch zur Immunstimulation im Sinne einer Behandlung der verletzten Haut, insbesondere zur Behandlung von Wunden.

Die Haut, insbesondere die Epidermis, ist als Barriereorgan des menschlichen Organismus in besonderem Maße äußeren Einwirkungen unterworfen. Nach dem heutigen wissenschaftlichen Verständnis repräsentiert die Haut ein immunologisches Organ, das als immunkompetentes peripheres Kompartiment eine eigene Rolle in induktiven, effektiven und regulativen Immunprozessen des Gesamtorganismen spielt.

Die Epidermis ist reich mit Nerven und Nervenendapparaten wie Vater-Pacini-Lamellenkörpern, Merkel-Zell-Neuritenkomplexen und freien Nervenendigungen für Schmerz-, Kälte-, Wärmeempfindung und Juckreiz ausgestattet.

Immunsuppression im allgemeinen ist die Unterdrückung oder Abschwächung der Reaktivität des Immunsystems. Die Immunsuppression kann in lokale und systemische Effekte aufgegliedert werden. Letztlich umfaßt sie eine Vielzahl verschiedenster Aspekte, welche alle eine Reduktion der normalen immunologischen Abwehrmechanismen der Haut beinhalten.

Viele mehr oder weniger empfindliche Personen haben auch bei Verwendung mancher desodorierenden oder antitranspirierend wirkenden Zubereitungen unter erythematösen Hauterscheinungen zu leiden

Erythematöse Hauterscheinungen treten auch als Begleiterscheinungen bei gewissen Hauterkrankungen oder -unregelmäßigkeiten auf. Beispielsweise ist der typische Hautausschlag beim Erscheinungsbild der Akne regelmäßig mehr oder weniger stark gerötet.

Hauptsächlich werden Antioxidantien als Schutzsubstanzen gegen den Verderb der sie enthaltenden Zubereitungen verwendet. Dennoch ist bekannt, daß auch in der menschlichen und tierischen Haut unerwünschte Oxidationsprozesse auftreten können. Solche Prozesse spielen eine wesentliche Rolle bei der Hautalterung.

Im Aufsatz "Skin Diseases Associated with Oxidative Injury" in "Oxidative Stress in Dermatology", S. 323 ff. (Marcel Decker Inc., New York, Basel, Hong Kong, Herausgeber: Jürgen Fuchs. Frankfurt, und Lester Packer, Berkeley/Californien), werden oxidative Schäden der Haut und ihre näheren Ursachen aufgeführt.

Soll menschliches Haar dauerhaft gefärbt werden, kommen in der Praxis lediglich oxidierende Haarfärbeverfahren in Betracht. Beim oxidativen Haartärben erfolgt die Ausbildung des Farbstoffchromophoren durch Reaktion von Präkursoren (Phenole, Aminophenole, seltener auch Diamine) und Basen (meistens p-Phenylendiamin) mit dem Oxidationsmittel, zumeist Wasserstoffperoxid. Wasserstoffperoxidkonzentrationen um 6% werden dabei gewöhnlich verwendet.

Üblicherweise wird davon ausgegangen, daß neben der Färbewirkung auch eine Bleichwirkung durch das Wasserstoffperoxid erfolgt. In oxidativ gefärbtem menschlichem Haar sind, ähnlich wie bei gebleichtem Haar, mikroskopische Löcher an den Stellen, an denen Melaningranula vorlagen, nachweisbar.

Tatsache ist, daß das Oxidationsmittel Wasserstoffperoxid nicht nur mit den Farbvorstufen, sondern auch mit der Haarsubstanz reagieren und dabei unter Umständen eine Schädigung des Haares bewirken kann.

Antioxidantien sind Substanzen, welche Oxidationsprozesse verhindern bzw. welche die Autoxidation ungesättigte Verbindungen enthaltender Fette verhindern. Antioxidantien. welche auch auf dem Gebiete der Kosmetik und der Pharmazie Verwendung finden, sind beispielsweise α-Tocopherol, insbesondere in Form des α-Tocopherylacetats, Sesamol, Gallensäurederivate, Butylhydroxyanisol und Butylhydroxytoluol.

Auch aus dem Grunde, solchen Reaktionen vorzubeugen, können kosmetischen Formulierungen zusätzlich Antioxidantien und/oder Radikalfänger einverleibt werden

Zwar sind einige Antioxidantien und Radikalfänger bekannt. So ist bereits in den US-Patentschriften 4,144,325 und 4,248,861 sowie aus zahlreichen anderen Dokumenten vorgeschlagen worden, Vitamin E, eine Substanz mit bekannter antioxidativer Wirkung in Lichtschutzformulierungen einzusetzen, dennoch bleibt auch hier die erzielte Wirkung weit hinter der erhofften zurück.

Eine Aufgabe der vorliegenden Erfindung war, die Nachteile des Standes der Technik zu beseitigen. Insbesondere sollten Wirkstoffe bzw. Zubereitungen, solche Wirkstoffe enthaltend, zur Verfügung gestellt werden, bei deren Verwendung die Schädigung der Haut und/oder des Haares durch oxidativen Einfluß zumindest gemindert, wenn nicht gänzlich verhindert werden können.

Eine weitere Aufgabe der vorliegenden Erfindung bestand darin, kosmetische Zubereitungen zur Verfügung zu stellen, welche vor oder nach Behandlung des Haars mit Haarfärbezubereitungen, selbst solcher mit einem Gehalt an starken Oxidationsmitteln wie z.B. Wasserstoffperoxid, deren schädigenden Oxidationswirkung entgegenwirken.

Insbesondere sollten Wirkstoffe und Zubereitungen, solche Wirkstoffe enthaltend, zur kosmetischen und dermatologischen Behandlung und/oder Prophylaxe erythematöser, entzündlicher, allergischer oder autoimmunreaktiver Erscheinungen, insbesondere Dermatosen, aber auch des Erscheinungsbildes des "Stingings" zur Verfügung gestellt werden.

Ferner sollten solche Wirkstoffe, bzw. Zubereitungen, solche Wirkstoffe enthaltend, zur Verfügung gestellt werden, welche zur Immunstimulation der Haut, dabei vorteilhaft auch zur Immunstimulation im Sinne der die Wundheilung fördernden Wirkung, verwendet werden können.

Schließlich war es Aufgabe der vorliegenden Erfindung, Herstellungsverfahren für solche Wirkstoffe zu konzipieren.

Es war überraschend und für den Fachmann nicht vorauszusehen, daß Tocopherylglycoside der allgemeinen Formel 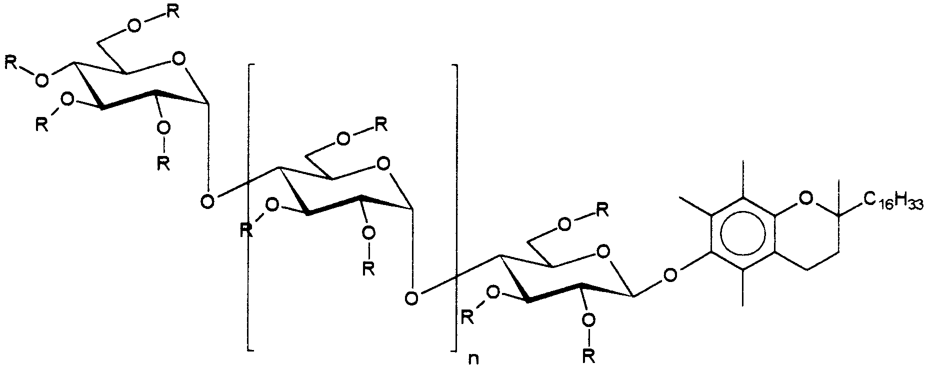 und/oder der allgemeinen Formel 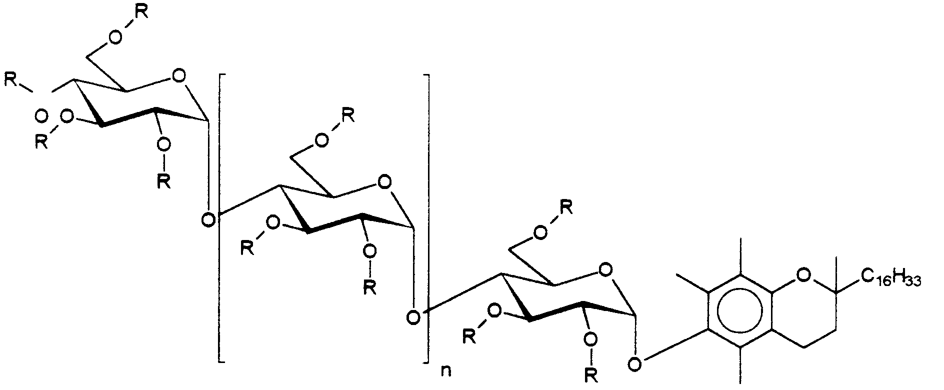 wobei n Werte von 0 - 6 annehmen kann und wobei R stellvertretend steht für die Gruppe bestehend aus den Resten H, verzweigtem und unverzweigtem Alkyl von 1 - 18 C-Atomen, verzweigtem und unverzweigtem Acyl von 1 - 18 C-Atomen, und wobei R innerhalb eines Moleküls in allen Positionen der Glycosylgruppen gleich sein kann, aber auch innerhalb eines Moleküls unterschiedliche Bedeutung annehmen kann, dergestalt, daß innerhalb eines Moleküls beliebige Kombinationen der stellvertretenen Reste gewählt werden dürfen,
all die geschilderten Nachteile des Standes der Technik beseitigen. Dabei ist es vorteilhart, die den erfindungsgemäßen Tocopheryglycosiden zugrundeliegende Tocopheroleinheit aus allen natürlichen oder synthetisch zugängigen Tocopherolkörpern zu wählen. Erfindungsgemäß besonders vorteilhaft ist, als zugrundeliegende Tocopheroleinheit DL-α-Tocopherol in seiner natürlichen Konfiguration zu wählen. Ganz besonders vorteilhart stellt R in allen Substitutionspositionen H dar.

Die bevorzugten Verbindungen tragen folgende Bezeichnungen:
n=0: Tocopheryl-β-D-maltosid bzw. Tocopheryl-α-D-maltosid
n= 1: Tocopheryl-β-D-maltotriosid bzw. Tocopheryl-α-D-maltotriosid
n= 2: Tocopheryl-β-D-maltotetraosid bzw. Tocopheryl-α-D-maltotetraosid
n= 3: Tocopheryl-β-D-maltopentaosid bzw. Tocopheryl-α-D-maltopentaosid
n= 4: Tocopheryl-β-D-maltohexaosid bzw. Tocopheryl-α-D-maltohexaosid
n= 5: Tocopheryl-β-D-maltoheptaosid bzw. Tocopheryl-α-D-maltohexaosid
n= 6: Tocopheryl-β-D-maltooctaosid bzw. Tocopheryl-α-D-maltooctaosid
n= 7: Tocopheryl-β-D-maltoenneaosid bzw. Tocopheryl-α-D-maltoenneaosid
n= 8: Tocopheryl-β-D-maltodekaosid bzw. Tocopheryl-α-D-maltodekaosid
Im allgemeinen sind sowohl die Tocophery-α-Glycoside als auch die Tocopheryl-β-Glycoside erfindungsgemäß vorteilhaft zu verwenden. Besonders vorteilhaft sind die β-Glycoside.

Auch Gemische aus α- und β-Glycosiden können erfindungsgemäß vorteilhart sein. Jedenfalls sind dem Fachmann eine Fülle von Verfahren bekannt, welche die Anomeren zu trennen imstande sind, beispielsweise chromatographische Verfahren.

Besonders vorteilhaft ist, wenn n den Wert 1 annimmt. Ganz besonders vorteilhaft stellt R in allen Substitutionspositionen H dar. Demgemäß ist das bevorzugte Tocopherylderivat durch die Strukturformel 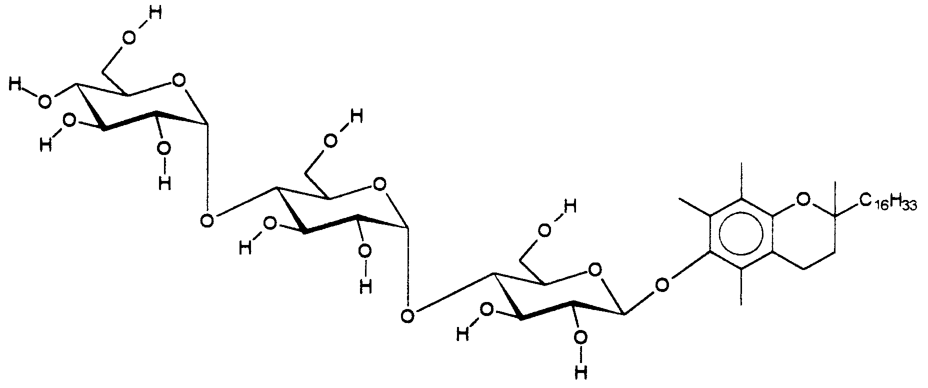 gekennzeichnet. Der C₁₆H₃₃₋ Rest ist durch die Strukturformel 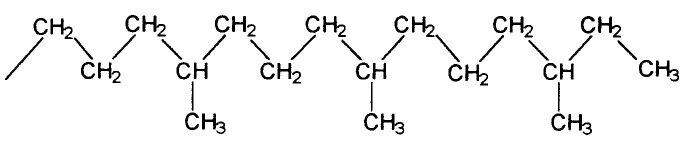 gekennzeichnet.

Es war für den Fachmann daher nicht vorauszusehen gewesen, daß die erfindungsgemäßen Tocopheryglycoside bzw. kosmetische oder dermatologische Zubereitungen, diese enthaltend
- besser als Antioxidans wirken
- besser als Radikalfänger wirken
- besser die Bindung von schädlichen Photoprodukten an Lipide, DNS und Proteine verhindern
- besser gegen die Hautalterung wirken
- besser die Haut gegen Photoreaktionen, insbesondere PLD, schützen
- besser entzündlichen Reaktionen vorbeugen würde
als die Wirkstoffe, Wirkstoffkombinationen und Zubereitungen des Standes der Technik und darüberhinaus
- gute Wasserdispergierbarkeit oder Wasserlöslichkeit besitzen
- vorzügliche Tensideigenschaften aufweisen
- in erstaunlicher Weise die Hautfeuchtigkeit erhöhen.

Die erfindungsgemäßen Tocopherylderivate sind imstande, die schädlichen Einflüsse von Sauerstoff auf die menschliche Haut zu minimieren, selbst bei gleichzeitiger Einwirkung von ultraviolettem Licht.

Erfindungsgemäß sind daher die Verwendung der erfindungsgemäßen Tocopherylglycoside als Antioxidantien sowie ihre Verwendung zur Bekämpfung und/oder Prophylaxe der durch oxidative Beanspruchung hervorgerufenen Hautalterung und entzündlicher Reaktionen.

Erfindungsgemäß sind auch Verfahren zur Herstellung von erfindungsgemäßen Tocopherylglycosiden, dadurch gekennzeichnet, daß ein Oligosaccharid oder ein Oligosaccharidderivat der allgemeinen Formel 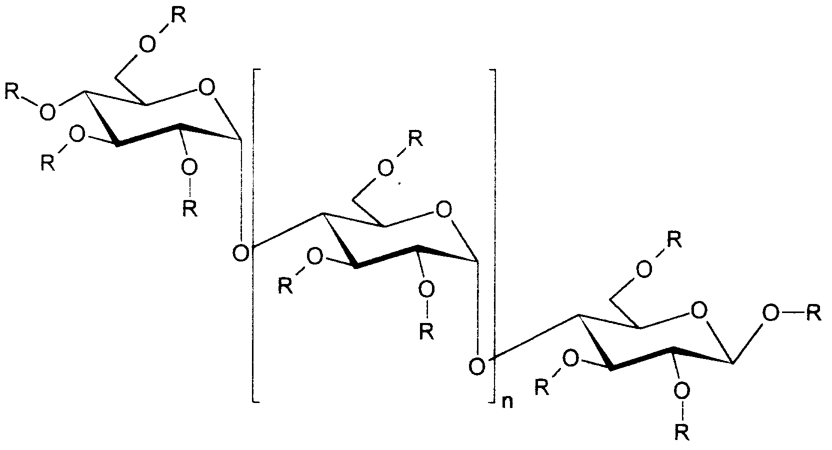 bzw. ein Oligosacchand oder ein Oligosaccharidderivat der allgemeinen Formel 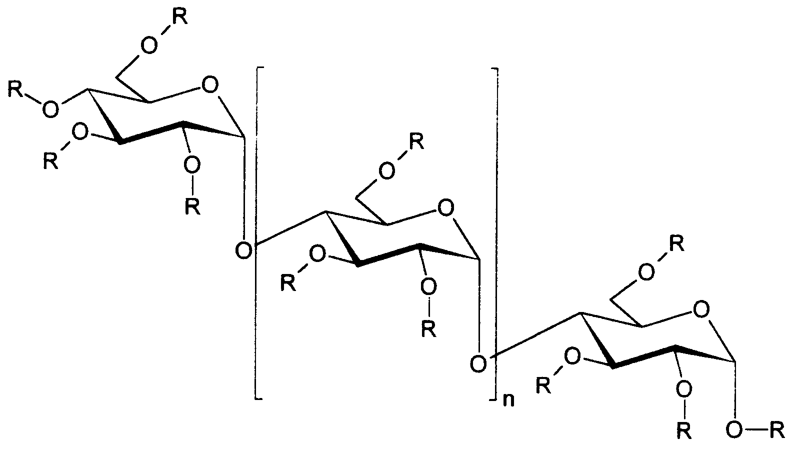 wobei n Werte von 0 - 6 annehmen kann und wobei R stellvertretend steht für die Gruppe bestehend aus den Resten H, verzweigtem und unverzweigtem Alkyl von 1 - 18 C-Atomen, verzweigtem und unverzweigtem Acyl von 1 - 18 C-Atomen, und wobei R innerhalb eines Moleküls in allen Positionen der Glycosylgruppen gleich sein kann, aber auch innerhalb eines Moleküls unterschiedliche Bedeutung annehmen kann, dergestalt, daß innerhalb eines Moleküls beliebige Kombinationen der stellvertretenen Reste gewählt werden dürfen, mit einem Tocopherolderivat der allgemeinen Formel 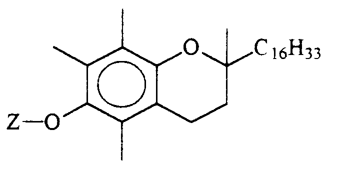 wobei Z bevorzugt ein Wasserstoffatom darstellt, zusammengeben wird und gegebenenfalls ein Protolyseschritt erfolgt, dergestalt, daß einer oder mehrere Reste R, welche nicht H darstellen, durch diesen Protolysesschritt gegen H ausgetauscht wird oder werden.

Bevorzugt ist ein Verfahren zur Herstellung von erfindungsgemäßen Tocopherylglycosiden, dadurch gekennzeichnet, daß das Oligosaccharid oder das Oligosaccharidderivat und das Tocopherolderivat in Gegenwart einer Lewissäure miteinander reagieren.

Insbesondere bevorzugt ist ein Verfahren zur Herstellung von erfindungsgemäßen Tocopherylglycosiden,dadurch gekennzeichnet, daß die Lewisbase gewählt wird aus der Gruppe BF₃ · Et₂O, SnCl₄, ZnCl₂.

Wird ein Protolysschritt gewünscht, dergestalt, daß einer oder mehrere Reste R, welche nicht H darstellen, durch diesen Protolysesschritt gegen H ausgetauscht wird oder werden, ist von Vorteil, wenn der Protolyseschritt darin besteht, daß ein Protonendonor sowie ein basisches Agens zugegeben wird.

Bevorzugt ist dann, daß das basische Agens gewählt wird aus der Gruppe der Alkalialkanolate, der Alkalicarbonate, der Amine.

Die Herstellung der erfindungsgemäßen Tocopherylglycoside erfolgt bevorzugt nach dem Reaktionsschema

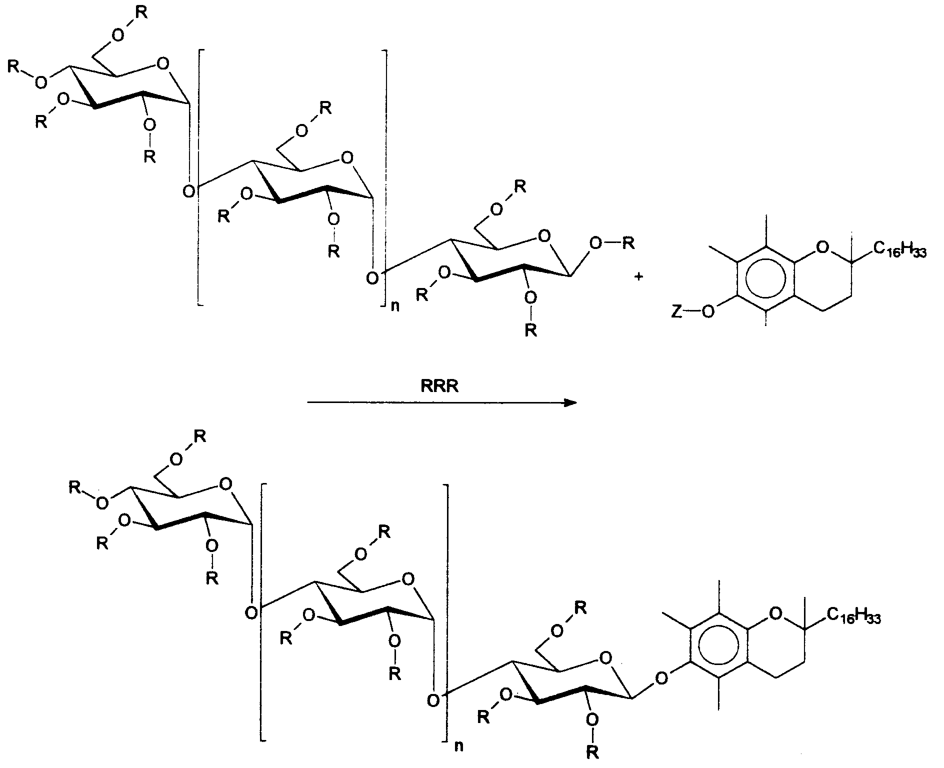

Der Rest Z ist vorteilhaft Wasserstoff. Dabei können die Reaktionsbedingungen RRR zwar grundsätzlich dem dem Fachmann geläufigen Wissensschatz entlehnt werden. Es ist aber von Vorteil und beruht auf eigenständiger erfinderischer Leistung, die hiermit offenbarten Bedingungen einzuhalten.

Vorstehend ist die Synthese eines β-Glycosids geschildert worden. Die Synthese eines α-Glycosids erfolgt vorteilhaft gemäß dem Reaktionsschema

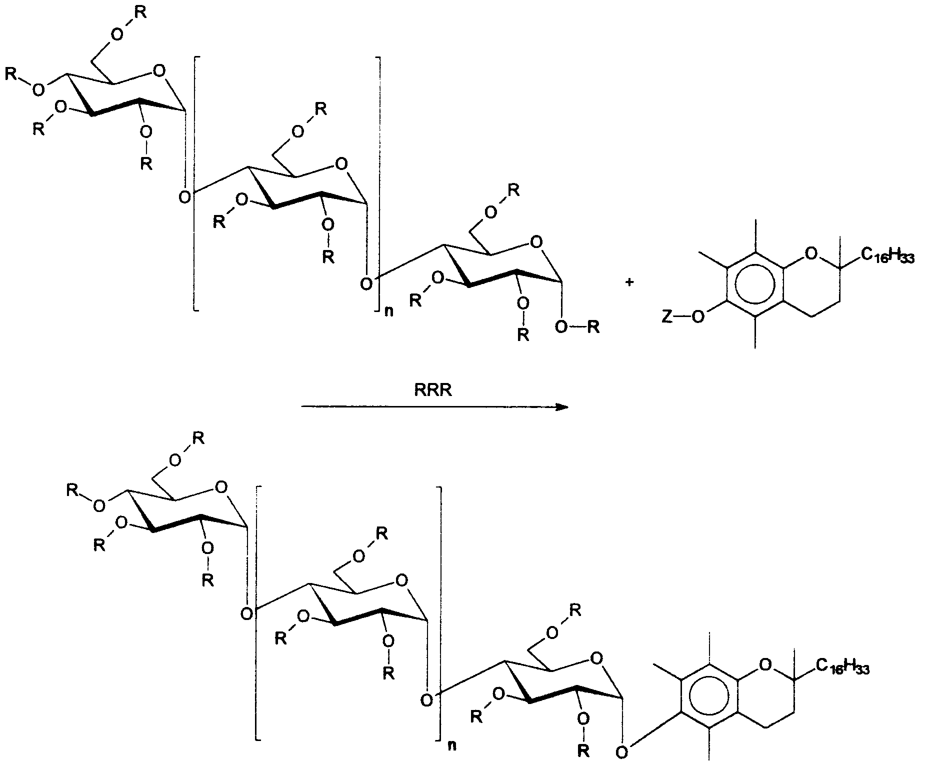

Die Rekationsbedingungen entsprechen mutatis mutandis denen der entsprechendenß-Glycoside.

Wenn R = H, ist es vorteilhaft, vor der Kopplung des Tocopherylrestes an das Oligoglycosid die OH-Gruppen mit Schutzgruppen, in den Strukturformeln und Reaktionsschemata mit der Bezeichnung ,,PG" bezeichnet, zu versehen:

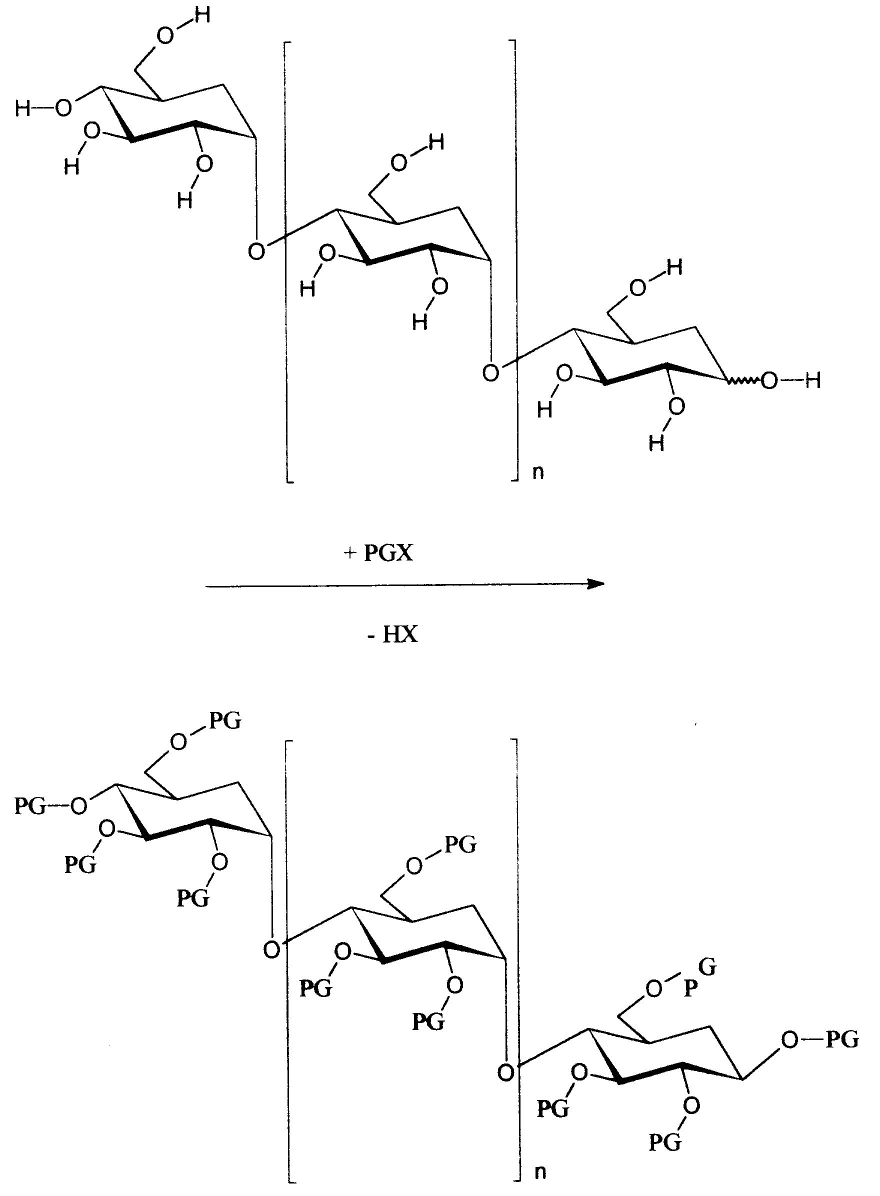

Solches Vorgehen ist dem Fachmann grundsätzlich bestens vertraut. Es hat sich allerdings als vorteilhaft herausgestellt, die OH-Gruppen der den erfindungsgemäßen Tocopherylglycosiden zugrundeliegenden Oligoglycoside mit Acetylresten zu schützen. Dies kann beispielsweise dadurch geschehen, daß das Oligoglycosid mit Essigsäureanhydrid und gewünschtenfalls Natriumacetat und/oder Zinkchlorid erhitzt wird, typischerweise in Zeiträumen von wenigen Minuten bis zu einigen Stunden, zum Beispiel 2 Stunden bei 120° C Bei dieser Reaktionsführung wird aus einem Anomerengemisch vorwiegend das Acetyl-ß-Glycosid gebildet.:

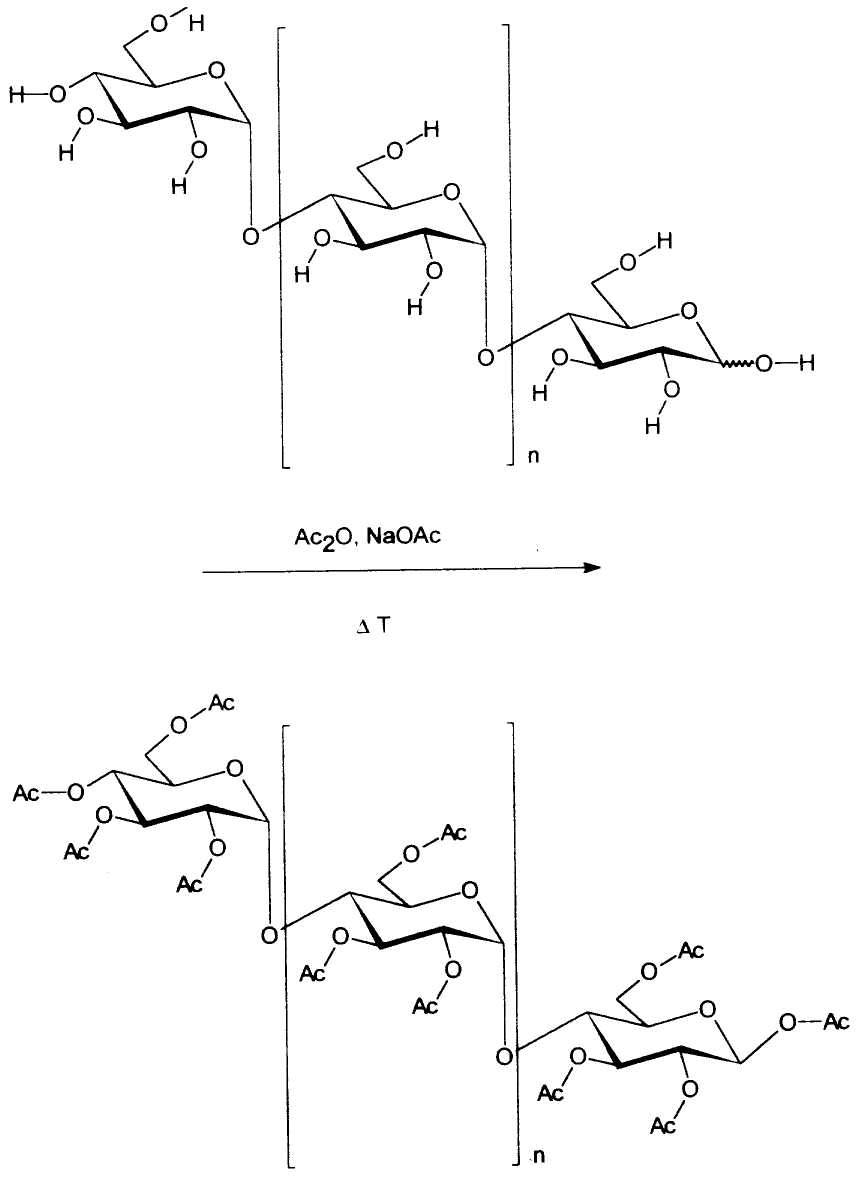

Das auf diese Weise geschützte Oligoglyscosid kann alsdann mit üblichen Glycosidierungshilfsmitteln mit dem Tocopherolgrundkörper vereinigt werden. Als vorteilhaftes Glycosidierungsmittel haben sich Lewissäuren, insbesondere BF₃ · Et₂O herausgestellt. Auch SnCl₄ und/oder ZnCl₂ sind vorteilhaft zu verwenden.

Demgemäß ist das bevorzugte Reaktionsschema

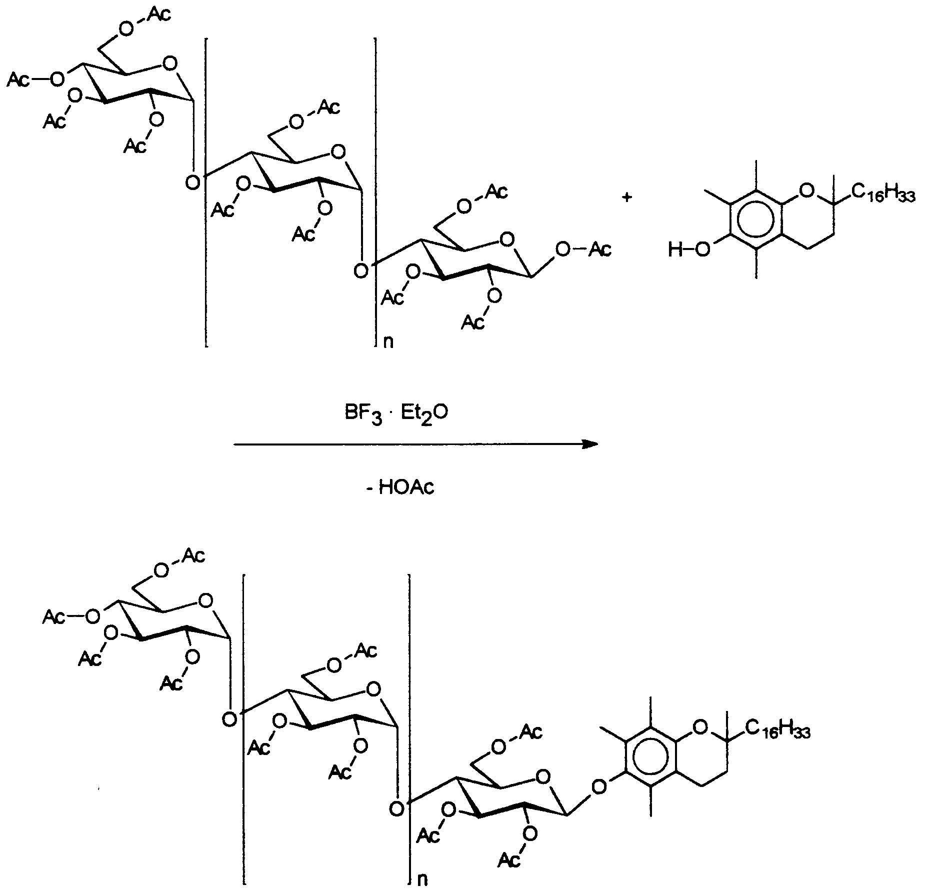

Vorteilhaft findet die vorstehende Reaktion unter zumindest weitgehendem, vorteilhaft vollständigem Wasserausschluß in organischen Lösungsmitteln wie z.B. Dichlormethan statt. Es ist von Vorteil, die Reaktion im Temperaturbereich von 0°C - 50°C durchzuführen, bevorzugt bei Raumtemperatur. Es kann vorteilhaft sein, unter Lichtausschluß zu arbeiten.

Es ist dem Fachmann natürlich klar, daß das entsprechende Tocopheryl-α-Glycosid aus dem entsprechenden Glycosid mit Acetatrest in α-Postition erzeugt werden kann.

Vorteilhaft ist weiter, die Glycosidierung unter weitgehendem, bevorzugt vollständigem Ausschluß von Sauerstoff bzw. anderer Oxidationsmittel auszuführen.

Es ist aber auch vorteilhaft, andere Glycosidierungsbedingungen zu wählen. Beispielsweise können enzymatische Prozesse angewandt werden. Auch das in der JP-OS Sho60-56994 beschriebene Verfahren hat sich als günstig erwiesen.

Sollen die Schutzgruppen abgespalten werden, stehen dem Fachmann die üblichen Hilfsmittel zur Verfügung. Beispielsweise ist vorteilhaft, Acetylgruppen im basischen Milieu abzuspalten, etwa nach dem Reaktionsschema:

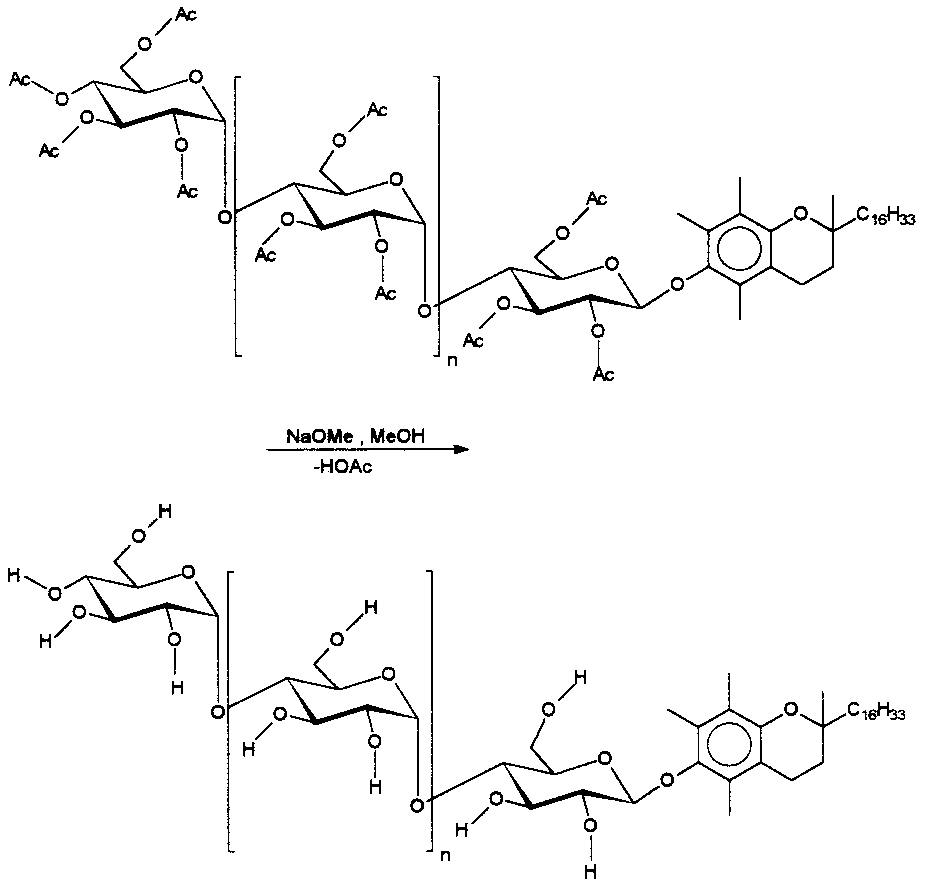

Als Basen können, wie im vorstehenden Schema, Alkalialkoholate verwendet werden, aber auch weniger starke Basen, z.B. K₂CO₃. Vorteilhaft ist auch, die Abspaltung der Schutzgruppen in eine Medium aus Triethylamin, Methanol und Wasser, bevorzugt in einem Verhältnis von etwa 1 : 8 : 1 vorzunehmen.

Die erfindungsgemäßen kosmetischen oder dematologischen Formulierungen können wie üblich zusammengesetzt sein und zur Behandlung, der Pflege und der Reinigung der Haut und/oder der Haare und als Schminkprodukt in der dekorativen Kosmetik dienen. Sie enthalten bevorzugt 0,001 Gew.-% bis 10 Gew.-%, insbesondere aber 0,01 Gew.-% bis 6 Gew.-%, bezogen auf das Gesamtgwicht der Mittels, an den erfindungsgemäßen Tocopheryglycosiden.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Erfindungsgemäße kosmetische und dermatologische Zubereitungen können in verschiedenen Formen vorliegen. So können sie z.B. eine Lösung, eine wasserfreie Zubereitung, eine Emulsion oder Mikroemulsion vorn Typ Wasser-in-Öl (W/O) oder vom Typ Öl-in-Wasser (O/W), eine multiple Emulsionen, beispielsweise vom Typ Wasser-in-Öl-in-Wasser (W/O/W), ein Gel, einen festen Stift, eine Salbe oder auch ein Aerosol darstellen. Es ist auch vorteilhaft, die erfindungsgemäßen Tocopheryglycoside in verkapselter Form darzureichen, z.B. in Kollagenmatrices und anderen üblichen Verkapselungsmaterialien, z.B. als Celluloseverkapselungen, in Gelatine, Wachsmatrices oder liposmal verkapselt. Insbesondere Wachsmatrices wie sie in der DE-OS 43 08 282 beschrieben werden, haben sich als günstig herausgestellt.

Es ist auch möglich und vorteilhaft im Sinne der vorliegenden Erfindung, die erfindungsgemäßen Tocopheryglycoside in wäßrige Systeme bzw. Tensidzubereitungen zur Reinigung der Haut und der Haare einzufügen.

Als vorteilhafte Verkörperung der vorliegenden Erfindung wird daher auch die Verwendung erfindungsgemäßer Tocopheryglycoside zum Schutze der Haut und/oder der Haare vor oxidativer Beanspruchung angesehen, insbesondere diese Verwendung der erfindungsgemäßen Tocopheryglycoside in Shampoos und Waschformulierungen.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, oberfiächenaktive Substanzen, Emulgatoren, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

Insbesondere können die erfindungsgemäßen Tocopheryglycoside auch mit anderen Antioxidantien kombiniert werden.

Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propyrthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-` Oleyl-, γ-Linoleyl-, Cholesteryl- und Glycerylester) sowie deren Satre, Dilaurylthiodipropionat, Distearyrthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure. Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmtat, Mg-Ascorbylphosphat, Ascorbylacatat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Hamsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zukker, Nukleotide, Nukleoside, Peptide und Lipide) dieser genannten Wirkstoffe.

Die Menge der vorgenannten Antioxidantien (eine oder mehrere Verbindungen), welche nicht mit den erfindungsgmäßen Tocopheryglycoside identisch sind, in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Erfindungsgemäße Emulsionen sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Die Lipidphase kann dabei vorteilhaft gewährt werden aus folgender Substanzgruppe:
- natürliche, synthetische und/oder partialsynthetische Öle, wie Triglyceride der Caprin- oder der Caprylsäure, vorzugsweise aber Rizinusöl;
- Fette, Wachse und andere natürliche synthetische und/oder partialsynthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Silikonöle wie Dimethylpolysiloxane, Diethylpolysiloxane, Diphenylpolysiloxane sowie Mischformen daraus.
- gesättigte Verbindungen wie Kohlenwasserstoffe natürlichen oder synthetischen Ursprungs (Vaseline, Squalan)

Die wäßrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl-oder -monobutylether, Propylenglykolmonomethyl, -monoethyl- oder -monobutylether, Diethylenglykolmonomethyl- oder -monoethylether und analoge Produkte sowie insbesondere ein oder mehrere Verdickungsmittel, welches oder welche vorteilhaft gewährt werden können aus der Gruppe Siliciumdioxid, Aluminiumsilikate, Polysaccharide bzw. deren Derivate, z.B. Hyaluronsäure, Xanthangummi, Hydroxypropylmethyicellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet. Bei alkoholischen Lösemitteln kann Wasser ein weiterer Bestandteil sein.

Erfindungsgemäße Emulsionen sind vorteilhaft und entharten z.B. die genannten Fette, Öle, Wachse und anderen Fettkörper, sowie Wasser und einen Emulgator, wie er üblicherweise für einen solchen Typ der Formulierung verwendet wird.

Gele gemäß der Erfindung enthatten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugsweise ein Polyacrylat ist.

Als Treibmittel für erfindungsgemäße, aus Aerosolbehältern versprühbare Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Enthaften die erfindungsgemäßen Emulsionen UVB-Fittersubstanzen, können diese öllöslich oder wasserlöslich sein. Erfindungsgemäß vorteilhafte öllösliche UVB-Filter sind z.B.:
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoësäureamylester;
- Ester der Zimtsäure, vorzugsweise 4-Methoxyzimtsäure(2-ethylhexyl)ester 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon:
- Ester der Benzalmalonsäure, vorzugsweise 4-Methoxybenzalmalonsäuredi(2-ethylhexyl)ester, - 2,4,6-Trianilino-(p-carbo-2'-ethyl-1'-hexyloxy) -1,3,5-triazin.

Vorteilhafte wasserlösliche UVB-Filter sind z.B.:
- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Die Liste der genannten UVB-Filter, die in Kombination mit den erfindungsgemäßen Tocopheryglycoside verwendet werden können, soll selbstverständlich nicht limitierend sein.

Gegenstand der Erfindung ist auch die Verwendung einer Kombination der erfindungsgemäßen Tocopheryglycoside mit mindestens einem UVB-Filter als Antioxidans bzw. die Verwendung einer Kombination der erfindungsgemäßen Tocopheryglycoside mit mindestens einem UVB-Filter als Antioxidans in einer kosmetischen oder dermatologischen Zubereitung.

Es kann auch von Vorteil sein, erfindungsgemäße Tocopheryglycoside mit UVA-Filtern zu kombinieren, die bisher üblicherweise in kosmetischen Zuberertungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch diese Kombinationen bzw. Zuberetungen, die diese Kombinationen enthalten, sind Gegenstand der Erfindung. Es können die für die UVB-Kombination verwendeten Mengen eingesetzt werden.

Gegenstand der Erfindung ist auch die Verwendung einer Kombination der erfindungsgemäßen Tocopheryglycoside mit mindestens einem UVA-Filter als Antioxidans bzw. die Verwendung einer Kombination der erfindungsgemäßen Tocopheryglycoside mit mindestens einem UVA-Filter als Antioxidans in einer kosmetischen oder dermatologischen Zubereitung.

Gegenstand der Erfindung ist auch die Verwendung einer Kombination der erfindungsgemäßen Tocopheryglycoside mit mindestens einem UVA-Filter und mindestens einem UVB-Filter als Antioxidans bzw. die Verwendung einer Kombination der erfindungsgemäßen Tocopheryglycoside mit mindestens einem UVA-Filter und mindestens einem UVB-Filter als Antioxidans in einer kosmetischen oder dermatologischen Zubereitung.

Kosmetische und dermatologische Zubereitungen mit einem wirksamen Gehalt an erfindungsgemäßen Tocopheryglycoside können auch anorganische Pigmente enthalten, die üblicherweise in der Kosmetik zum Schutze der Haut vor UV-Strahlen verwendet werden. Dabei handelt es sich um Oxide des Titans, Zinks Eisens, Zirkoniums, Siliciums, Mangans, Aluminiums, Cers und Mischungen davon, sowie Abwandlungen, bei denen die Oxide die aktiven Agenten sind. Besonders bevorzugt handelt es sich um Pigmente auf der Basis von Titandioxid.

Auch diese Kombinationen von UVA-Filter und Pigment bzw. Zubereitungen, die diese Kombination enthalten, sind Gegenstand der Erfindung. Es können die für die vorstehenden Kombinationen genannten Mengen verwendet werden.

Bei kosmetischen und dermatologischen Zubereitungen zum Schutze der Haare vor UV-Strahlen gemäß der Erfindung handelt es sich beispielsweise um Shampoonierungsmittel, Zubereitungen, die beim Spülen der Haare vor oder nach der Shampoonierung, vor oder nach der Dauerwellbehandlung, vor oder nach der Färbung oder Entfärbung der Haare angewendet werden, um Zubereitungen zum Fönen oder Einlegen der Haare, Zuberetungen zum Färben oder Entfärben, um eine Frisier- und Behandlungslotion, einen Haarlack oder um Dauerwellmittel.

Die kosmetischen und dermatologischen enthalten Wirkstoffe und Hilfsstoffe, wie sie üblicherweise für diesen Typ von Zubereitungen zur Haarpflege und Haarbehandlung verwendet werden. Als Hilfsstoffe dienen Konservierungsmittel, oberflächenaktive Substanzen, Substanzen zum Verhindern des Schäumens, Verdickungsmittel, Emulgatoren, Fette, Öle, Wachse, organische Lösungsmittel. Bakterizide, Parfüme, Farbstoffe oder Pigmente, deren Aufgabe es ist, die Haare oder die kosmetische oder dermatologische Zubereitung selbst zu färben, Elektroyte, Substanzen gegen das Fetten der Haare.

Unter Elektrolyten im Sinne der vorliegenden Erfindung sind wasserlösliche Alkali-, Ammonium-, Erdalkali- (unter Einbeziehung des Magnesiums) und Zinksalze anorganischer Anionen und beliebige Gemische aus solchen Salzen zu verstehen, wobei gewährleistet sein muß, daß sich diese Salze durch pharmazeutische oder kosmetische Unbedenklichkeit auszeichnen.

Die erfindungsgemäßen Anionen werden bevorzugt gewährt aus der Gruppe der Chlorde, der Sulfate und Hydrogensulfate, der Phosphate Hydrogenphosphate und der linearen und cyclischen Oligophosphate sowie der Carbonate und Hydrogencarbonate.

Kosmetische Zubereitungen, die ein Hautreinigungsmittel oder Shampoonierungsmittel darstellen, enthalten vorzugsweise mindestens eine anionische, nicht-ionische oder amphotere oberflächenaktive Substanz, oder auch Gemische aus solchen Substanzen, erfindungsgemäße Tocopheryglycoside im wäßrigen Medium und Hilfsmittel, wie sie üblicherweise dafür verwendet werden. Die oberflächenaktive Substanz bzw. die Gemische aus diesen Substanzen können in einer Konzentration zwischen 1 Gew.-% und 50 Gew.-% in dem Shampoonierungsmittel vorliegen.

Liegen die kosmetischen oder dermatologischen Zubereitungen in Form einer Lotion vor, die ausgespült und z.B. vor oder nach der Entfärbung, vor oder nach der Shampoonierung, zwischen zwei Shampoonierungsschritten, vor oder nach der Dauerwellbehandlung angewendet wird, so handelt es sich dabei z.B. um wäßrige oder wäßrig-alkoholische Lösungen, die gegebenenfalls oberflächenaktive Substanzen enthalten, deren Konzentration zwischen 0,1 und 10 Gew.-%, vorzugsweise zwischen 0,2 und 5 Gew.-%, liegen kann.

Diese kosmetischen oder dermatologischen Zubereitungen können auch Aerosole mit den üblicherweise dafür verwendeten Hilfsmitteln darstellen.

Eine kosmetische Zubereitung in Form einer Lotion, die nicht ausgespült wird, insbesondere eine Lotion zum Einlegen der Haare, eine Lotion, die beim Fönen der Haare verwendet wird, eine Frisier- und Behandlungslotion, stellt im allgemeinen eine wäßrige, alkoholische oder wäßrig-alkoholische Lösung dar und enthält mindestens ein kationisches, anionisches, nicht-ionisches oder amphoteres Polymer oder auch Gemische derselben, sowie erfindungsgemäße Wirkstoffkombinatonen in wirksamer Konzentration. Die Menge der verwendeten Polymeren liegt z.B. zwischen 0,1 und 10 Gew -%, bevorzugt zwischen 0,1 und 3 Gew.-%.

Kosmetische Zubereitungen zur Behandlung und Pflege der Haare, die erfindungsgemäße Tocopheryglycoside enthalten, können als Emulsionen vorliegen, die vorn nichtionischen oder anionischen Typ sind. Nicht-ionische Emulsionen enthalten neben Wasser Öle oder Fettalkohole, die beispielsweise auch polyethoxyliert oder polypropoxyliert sein können oder auch Gemische aus den beiden organischen Komponenten. Diese Emulsionen enthalten gegebenenfalls kationische oberflächenaktive Substanzen.

Erfindungsgemäß können kosmetische Zubereitungen zur Behandlung und Pflege der Haare als Gele vorliegen, die neben einem wirksamen Gehalt an erfindungsgemäßen Tocopheryglycoside und dafür üblicherweise verwendeten Lösungsmitteln, bevorzugt Wasser, noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdikkungsmittel, z.B. Aluminiumsilikate wie beispielsweise Bentonite, oder ein Gemisch aus Polyethylenglykol und Polyethylenglycolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Vorzugsweise beträgt die Menge der erfindungsgemäßen Tocopheryglycoside in einem für die Haare bestimmten Mittel 0,05 Gew.-% bis 10 Gew.-%, insbesondere 0,5 Gew.-% bis 5 Gew.-%, bezogen auf das Gesamtgewicht des Mittels.

Erfindungsgemäße wäßrige kosmetische Reinigungsmittel oder für die wäßrige Reinigung bestimmte wasserarme oder wasserfreie Reinigungsmittelkonzentrate können anionische, nichtionische und/oder amphotere Tenside enthalten, beispielsweise
- herkömmliche Seifen, z.B. Fettsäuresalze des Natriums
- Alkylsulfate, Alkylethersulfate, Alkan- und Alkylbenzolsulfonate
- Sulfoacetate
- Sulfobetaine
- Sarcosinate
- Amidosulfobetaine
- Sulfosuccinate
- Sulfobernsteinsäurehalbester
- Alkylethercarboxylate
- Eiweiß-Fettsäure-Kondensate
- Alkylbetaine und Amidobetaine
- Feffsäurealkanolamide
- Polyglycolether-Derivate

Kosmetische Zubereitungen, die kosmetische Reinigungszubereitungen für die Haut darstellen, können in flüssiger oder fester Form vorliegen. Sie enthalten neben erfindungsgemäßen Tocopheryglycoside vorzugsweise mindestens eine anionische, nichtionische oder amphotere oberflächenaktive Substanz oder Gemische daraus, gewünschtenfalls einen oder mehrere Elektrolyten und Hilfsmittel, wie sie üblicherweise dafür verwendet werden. Die oberflächenaktive Substanz kann in einer Konzentration zwischen 1 und 94 Gew.-% in den Reinigungszubereitungen vorliegen, bezogen auf das Gesamtgewicht der Zubereitungen.

Kosmetische Zubereitungen, die ein Shampoonierungsmittel darstellen, enthalten neben einem wirksamen Gehalt an erfindungsgemäßen Tocopheryglycoside vorzugsweise mindestens eine anionische, nicht-ionische oder amphotere oberflächenaktive Substanz oder Gemische daraus, gegebenenfalls einen erfindungsgemäßes Elektrolyten und Hilfsmittel, wie sie üblicherweise dafür verwendet werden. Die oberflächenaktive Substanz kann in einer Konzentration zwischen 1 Gew.-% und 94 Gew.-% in dem Shampoonierungsmittel vorliegen.

Die erfindungsgermäßen Zusammensetzungen enthalten außer den vorgenannten Tensiden Wasser und gegebenenfalls die in der Kosmetik üblichen Zusatzstoffe, beispielsweise Parfüm, Verdicker, Farbstoffe, Desodorantien, antimikrobielle Stoffe, rückfettende Agentien, Komplexierungs- und Sequestrierungsagentien, Perlglanzagentien, Pflanzenextrakte Vitamine, Wirkstoffe und dergleichen.

Die vorliegende Erfindung umfaßt auch ein kosmetisches Verfahren zum Schutze der Haut und der Haare vor oxidativen bzw. photooxidativen Prozessen, das dadurch gekennzeichnet ist, daß man ein kosmetisches Mittel, welches eine wirksame Konzentration an erfindungsgemäßen Tocopheryglycoside enthält, in ausreichender Menge auf die Haut oder Haare aufbringt.

Ebenso umfaßt die vorliegende Erfindung auch ein Verfahren zum Schutze kosmetischer oder dermatologischer Zubereitungen gegen Oxidation oder Photooxidation, wobei diese Zuberertungen z.B. Zubereitungen zur Behandlung und Pflege der Haare darstellen, insbesondere Haarfärbemittel, Haarlacke, Shampoonierungsmittel, Farbshampoonierungsmittel, ferner Schminkprodukte wie z.B. Nagellacke, Lippenstifte, Teintgrundlagen, Wasch- und Duschzubereitungen, Cremes zur Behandlung oder Pflege der Haut oder um sämtliche anderen kosmetischen Zubereitungen handelt, deren Bestandteile Stabilitätsprobleme aufgrund von Oxidation bzw. Photooxidation bei der Lagerung mit sich bringen können, dadurch gekennzeichnet, daß die kosmetischen Zubereitungen einen wirksamen Gehalt an erfindungsgemäßen Tocopheryglycoside aufweisen.

Vorzugsweise beträgt die Menge an erfindungsgemäßen Tocopheryglycoside in diesen Zubereitungen 0,001 Gew.-% bis 10 Gew.-%, insbesondere 0,1 Gew.-% bis 3 Gew.-%, bezogen auf das Gesamtgewicht der Zuberertungen.

Gegenstand der Erfindung ist auch das Verfahren zur Herstellung der erfindungsgemäßen kosmetischen Mittel, das dadurch gekennzeichnet ist, daß man in an sich bekannter Weise erfindungsgemäßen Tocopheryglycoside in kosmetische und dermatologische Formulierungen einarbeitet.

Die nachfolgenden Beispiele sollen die vorliegende Erfindung verdeutlichen, ohne sie einzuschränken. Die in den Beispielen 1 - 3 geschilderte Herstellung der Peracetate der Oligosaccharide, der Peracetate der Tocopherylglycoside und letztendlich der Tocopherylglycoside selbst läßt sich mutatis mutandis auf sämtliche erfindungsgemäßen Tocopherylglycoside und deren Vorstufen anwenden.

### Beispiel 1

Acetylierung: 1 mmol Maltotriose wird zu einer auf 120° C erwärmten Suspension aus 2 mmol Natriumacetat und Essigsäureanhydrid (je mmol OH-Gruppe der Maltotriose: 4 mmol Essigsäureanhydrid) gegeben. Die Temperatur des Reaktionsansatzes wird etwa 2 Stunden auf ca. 120° C gehalten. Anschließend wird die Reaktionsmischung zur Hydrolyse des Essigsäureanhydrids mit Eiswasser versetzt (auf 10 ml). Es werden 3 ml Dichlormethan zugegeben und 12 Stunden bei Raumtemperatur gerührt. Die wäßrige Phase wird abgetrennt und dreimal mit je 10 ml Dichlormethan extrahiert. Die vereinigten organischen Phasen werden mit gesättigter Natriumhydroxidlösung neutralisiert, zweimal mit 10 ml Wasser gewaschen und über Magnesiumsulfat getrocknet. Das Lösungsmittel wird im Vakuum entfernt.

Ausbeute: 80 % Maltotrioseperacetat

### Beispiel 2

0,6 mmol nach Beispiel 1 erhaltenen Maltotrioseperacetats und 3,6 mmol α-Tocopherol werden mit 1,44 mmol BF₃· Et₂O in wasserfreiem Dichlormethan gelöst und bei Raumtemperatur unter Lichtausschluß gerührt. Die Reaktion wird nach drei Stunden abgebrochen. Die Reaktionsmischung wird mit gesättigter Natriumhydrogencarbonatlösung neutralisiert, dreimal mit Wasser gewaschen, über Magnesiumsulfat getrocknet. Das Lösungsmittel wird im Vakuum entfernt. Nach flash-chromatographischer Reinigung (Laufmittel: Dichlormethan/Aceton im Verhältnis 17 : 1) erhält man ein gelbliches Glas.

Ausbeute: 69 % peracetyliertes Tocopherylmaltotriosid

### Beispiel 3

0,121 mmol des in Beispiel 2 erhaltenen peracetylierten Tocopherylmaltotriosids werden in sauerstofffreiem absolutem Methanol gelöst, welches mit frisch zubereitetem Natriummethanolat bis zum Erreichen eines pH-Wertes von 8 versetzt wurde. Nach vollständiger Umsetzung wird mit Ionenaustauscher (Amberlite 120 H⁺) neutralisiert. Die Reaktionslösung wird bei vermindertem Druck im Rotationsverdampfer eingeengt und im Vakuum getrocknet.

Ausbeute: 77 % Tocopheryl-β-D-maltotriosid

### Beispiel 4

| W/O-Emulsion | |
|---|---|
| | Gew.-% |
| DL-α-Tocopheryl-β-D-matotriosid | 2,00 |
| Polyglyceryl-3-diisostearat | 2,50 |
| Paraffinwachs | 3,00 |
| Paraffinöl DAB 9 | 10,00 |
| Cetearyloctanoat | 10,00 |
| Bienenwachs | 4,00 |
| Glycerin | 5,00 |
| Parfum, Konservierungsmittel, Zusatzstoffe | q.s. |
| Wasser, demin. | ad 100,00 |

### Beispiel 5

| W/O-Emulsion | |
|---|---|
| | Gew.-% |
| DL-α-Tocopheryl-β-D-maltotriosid | 0,50 |
| DL-α-Tocopheryl-β-D-maltotetraosid | 0,50 |
| Cholesterin | 1,50 |
| Paraffinwachs | 3,00 |
| Vaseline | 5,00 |
| Paraffinöl DAB 9 | 20,00 |
| Glycerin | 5,00 |
| Parfum, Konservierungmittel, Zusatzstoffe | q.s. |
| Wasser | ad 100,00 |

### Beispiel 6

| W/O-Emulsion | |
|---|---|
| | Gew.-% |
| DL-α-Tocopheryl-β-D-maltotriosid | 1,00 |
| Cholesterin | 1,50 |
| Paraffinwachs | 3,00 |
| Vaseline | 5,00 |
| Paraffinöl DAB 9 | 20,00 |
| Glycerin | 5,00 |
| Parfum, Konservierungsmittel, Zusatzstoffe | q.s. |
| Wasser | ad 100,00 |

### Beispiel 7

| W/O-Emulsion | |
|---|---|
| | Gew.-% |
| DL-α-Tocopheryl-β-D-maltotriosid | 0,50 |
| Wollwachsalkohole | 2,50 |
| Paraffinwachs | 6,00 |
| Bienenwachs | 1,00 |
| Vaseline | 3,00 |
| Pamffinöl DAB 9 | 20,00 |
| Glycerin | 5,00 |
| Parfum, Konservierungsmittel, Zusatzstoffe | q.s. |
| Wasser | ad 100,00 |

### Beispiel 8

| O/W-Emulsion | |
|---|---|
| | Gew.-% |
| DL-α-Tocopheryl-β-D-maltotetraosid | 1,00 |
| Sorbitanstearat | 2,50 |
| Vaseline | 2,50 |
| Paraffinöl DAB 9 | 14,00 |
| Hydrierte Kokosfettsäureglyceride | 1,00 |
| Carbomer 934 | 0,20 |
| 1,3-Butylenglycol | 2,00 |
| Parfum, Konservierungsmittel, Farbstoffe, Zusatzstoffe | q.s. |
| Wasser demin | ad 100,00 |

### Beispiel 9

| O/W-Emulsion | |
|---|---|
| | Gew.-% |
| DL-α-Tocopheryl-β-D-maltohexaosid | 0,20 |
| Glycerylmonostearat | 0,50 |
| Stearinsäure | 2,50 |
| Vaseline | 3,00 |
| Paraffinöl DAB 9 | 15,00 |
| Carbomer 934 | 0,20 |
| Cetylphosphat | 0,15 |
| Glycerin | 3,00 |
| Dimethicone | 0,50 |
| Parfum, Konservierungsmittel, Farbstoffe, Zusatzstoffe | q.s. |
| Wasser VES | ad 100,00 |

### Beispiel 10

| O/W-Emulsion | |
|---|---|
| | Gew.-% |
| DL-α-Tocopheryl-β-D-maltohexaosid | 0,40 |
| Glycerylmonostearatcitrat | 1,00 |
| Sorbitanstearat | 1,00 |
| Vaseline | 1,00 |
| Paraffinöl DAB 9 | 15,00 |
| Hydrierte Kokosfettsäureglyceride | 1,00 |
| Carbomer 934 | 0,20 |
| Cetylphosphat | 0,10 |
| Glycerin | 3,00 |
| Parfum, Konservierungsmittel, Farbstoffe, Zusatzstoffe | q.s. |
| Wasser demin. | ad 100,00 |

### Beispiel 11

| O/W-Emulsion | |
|---|---|
| | Gew.-% |
| DL-α-Tocopheryl-β-D-maltotetraosid | 0,50 |
| Glycerylstearatcitrat | 2,00 |
| Vaseline | 4,00 |
| Paraffinöl DAB 9 | 11,00 |
| Hydrierte Kokosfettsäureglyceride | 3,00 |
| Carbomer 934 | 0,20 |
| Glycerin | 3,00 |
| Dimethicone | 1,00 |
| Parfum, Konservierungsmittel, Farbstoffe, Zusatzstoffe | q.s. |
| Wasser demin. | ad 100,00 |

### Beispiel 12

| O/W-Emulsion | |
|---|---|
| | Gew.-% |
| DL-α-Tocopheryl-β-D-maltotetraosid | 1,00 |
| Sorbitanstearat | 2,50 |
| Vaseline | 2,50 |
| Paraffinöl DAB 9 | 14,00 |
| Hydrierte Kokosfettsäureglyceride | 1,00 |
| Carbomer 934 | 0,20 |
| Cetylphosphat | 0,15 |
| Glycerin | 3,00 |
| 1,3-Butylenglycol | 2,00 |
| Octylmethoxycinnamat | 2,00 |
| Parfum, Konservierungsmittel, Farbstoffe, Zusatzstoffe | q.s. |
| Wasser demin. | ad 100,00 |

### Beispiel 13

| O/W-Emulsion | |
|---|---|
| | Gew.-% |
| DL-α-Tocopheryl-β-D-matotriosid | 0,50 |
| DL-α-Tocopheryl-β-D-maltotetraosid | 3,50 |
| DL-α-Tocopheryl-β-D-maltohexaosid | 0,50 |
| Vaseline | 1,00 |
| Paraffinöl DAB 9 | 15,00 |
| Carbomer 934 | 0,20 |
| Cetylphosphat | 0,15 |
| Glycerin | 5,00 |
| Parfum, Konservierungsmittel, Farbstoffe, Zusatzstoffe | q.s. |
| Wasser demin. | ad 100,00 |

Mit NaOH kann der pH-Wert der in den Beispielen genannten Formulierungen, insbesondere der O/W-Emulsionen, vorteilhaft auf Werte im Bereiche von 5,0 - 8,0 eingestellt werden.

## Patentansprüche

1. Tocopherylglycoside der allgemeinen Formel 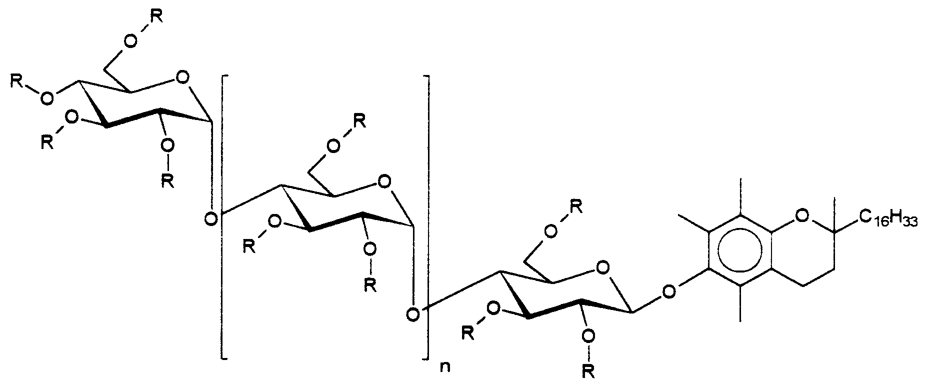 oder der allgemeinen Formel 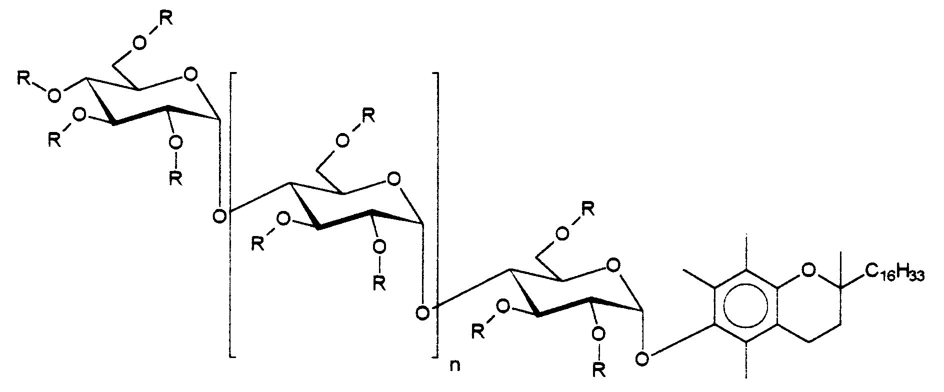 wobei n Werte von 0 - 6 annehmen kann und wobei R stellvertretend steht für die Gruppe bestehend aus den Resten H, verzweigtem und unverzweigtem Alkyl von 1 - 18 C-Atomen, verzweigtem und unverzweigtem Acyl von 1 - 18 C-Atomen, und wobei R innerhalb eines Moleküls in allen Positionen der Glycosylgruppen gleich sein kann, aber auch innerhalb eines Moleküls unterschiedliche Bedeutung annehmen kann, dergestalt, daß innerhalb eines Moleküls beliebige Kombinationen der stellvertretenden Reste gewählt werden dürfen.

2. Tocopherylglycoside nach Anspruch 1, gewählt aus der Gruppe Tocopheryl-β-D-maltosid, Tocopheryl-β-D-maltotriosod, Tocopheryl-β-D-maltotetrasid, Tocopheryl-β-D-maltopentaosid, Tocopheryl-β-D-maltohexaosid, Tocopheryl-β-D-maltoheptaosid, Tocopheryl-β-D-maltooctaosid, Tocopheryl-β-D-maltoenneaosid, Tocopheryl-β-D-maltodeakosid.

3. Verfahren zur Herstellung von Tocopherylglycosiden nach Anspruch 1 oder 2, dadurch gekennzeichnet, daß ein Oligosacharid oder ein Oligosacharidderivat der allgemeinen Formel 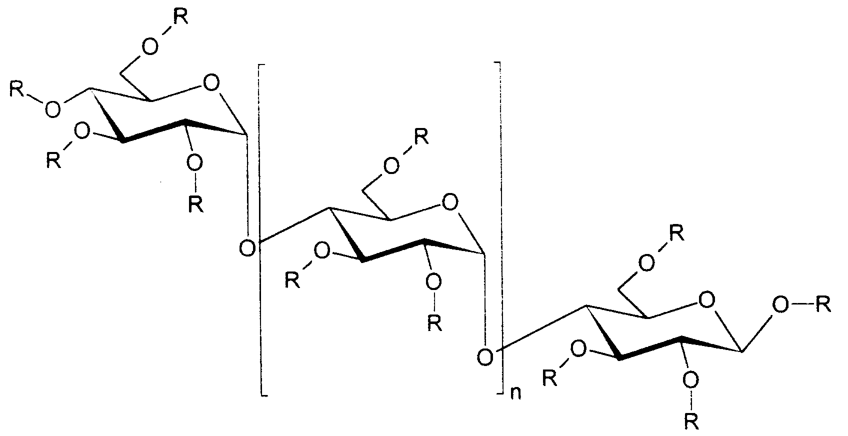 bzw, ein Oligosaccharid oder ein Oligosaccharidderivat der allgemeinen Formel 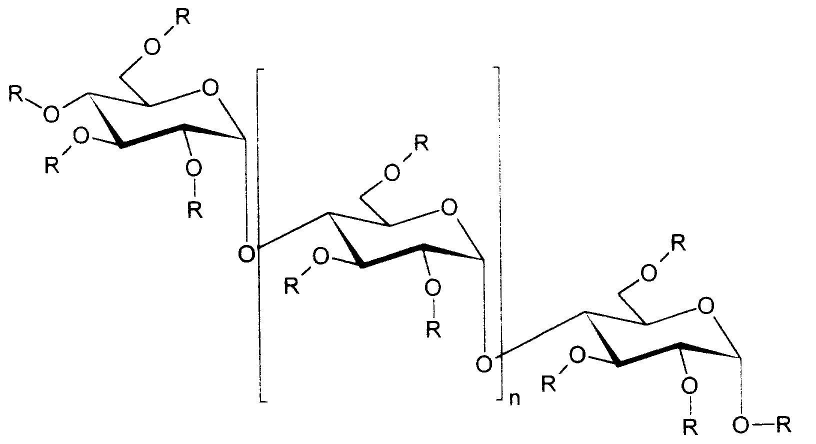 wobei n Werte von 0 - 6 annehmen kann und wobei R stellvertretend steht für die Gruppe bestehend aus den Resten H, verzweigtem und unverzweigtem Alkyl von 1 - 18 C-Atomen, verzweigtem und unverzweigtem Acyl von 1 - 18 C-Atomen, und wobei R innerhalb eines Moleküls in allen Positionen der Glycosylgruppen gleich sein kann, aber auch innerhalb eines Moleküls unterschiedliche Bedeutung annehmen kann, dergestalt, daß innerhalb eines Moleküls beliebige Kombinationen der stellvertretenen Reste gewählt werden dürfen, mit einem Tocopherolderivat der allgemeinen Formel 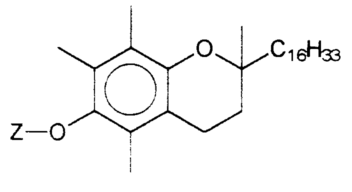 wobei Z bevorzugt ein Wasserstoffatom darstellt, zusammengeben wird und gegebenenfalls ein Protolyseschritt erfolgt, dergestallt, daß einer oder mehrere Reste R, welche nicht H darstellen, durch diesen Protolysesschritt gegen H ausgetauscht wird oder werden.

4. Verfahren nach Anspruch 3, dadurch gekennzeichnet, daß das Oligosaccharid oder das Oligosaccharidderivat und das Tocopherolderivat in Gegenwart einer Lewissäure miteinander reagieren.

5. Verfahren nach Anspruch 4, dadurch gekennzeichnet, daß die Lewisbase gewählt wird aus der Gruppe BF₃ · Et₂O, SnCl₄, ZnCl₂.

6. Verfahren nach Anspruch 3, dadurch gekennzeichnet, daß der Protolyseschritt darin besteht, daß ein Protonendonor sowie ein basisches Agens zugegeben wird.

7. Verfahren nach Anspruch 6, dadurch gekennzeichnet, daß das basische Agens gewählt wird aus der Gruppe der Alkalialkanolate, der Alkalicarbonate, der Amine.

8. Verwendung von Tocopherolderivaten nach Anspruch 1 als Antioxidantien.

9. Verwendung von Tocopherolderivaten nach Anspruch 1 zur kosmetischen oder pharmazeutischen Behandlung und/oder Prophylaxe von durch Oxidationsprozessen hervorgerufenen Hautveränderungen.
